# EUROPEAN PATENT APPLICATION

(11) **EP 4 706 588 A1**
(43) Date of publication of application: **11.03.2026**
(21) Application number: 25197975.3
(22) Date of filing: 25.08.2025
(51) Int. Cl.: A61C 9/00, A61C 19/04

(54) **METHOD AND APPARATUS FOR EXAMINING BONE FORMATION OF ALVEOLAR BONE**

(30) Priority: 05.09.2024 KR 20240120775
(71) Applicant: Huvitz Co., Ltd., Anyang-si, Gyeonggi-do 14055 (KR)
(72) Inventor: LEE, Weon Joon, 14055 Anyang-si (KR); HAN, Su Min, 14055 Anyang-si (KR)
(74) Representative: Henkel & Partner mbB

(57) **Abstract**

Disclosed are a method and apparatus for examining bone formation of an alveolar bone by using an intraoral scanner having a tomographic function. The method for examining bone formation of an alveolar bone is a method of examining bone formation of an alveolar bone using an intraoral scanner having a tomographic function, wherein the intraoral scanner includes a surface shape acquisition optical system (12 and 14) for obtaining a surface shape image of an oral structure (S) and an internal cross-section acquisition optical system (22 and 24) for obtaining an internal cross-sectional image of the oral structure (S), and the method includes filling an alveolar bone (2) requiring bone grafting with a bone grafting material (4), suturing gums (6) so as to cover the bone grafting material (4), and then obtaining a surface shape image of a part where an alveolar bone is formed using the surface shape acquisition optical system (12 and 14); obtaining an internal cross-sectional image showing a state of the alveolar bone (2) and the bone grafting material (4) by using the internal cross-section acquisition optical system (22 and 24) at a predetermined location (6a) of the obtained surface shape image; obtaining an internal cross-sectional image showing the state of the alveolar bone (2) and the bone grafting material (4) at a corresponding location by using the internal cross-section acquisition optical system (22 and 24) of the intraoral scanner when a gum surface shape at the location (6a) where the internal cross-sectional image was previously obtained is detected while obtaining gum surface shapes by moving the intraoral scanner after a predetermined period has elapsed; and comparing the internal cross-sectional images obtained at a predetermined time interval.

## Description

### CROSS-REFERENCE TO RELATED APPLICATION

This application claims benefit of priority to Korean Patent Application No. 10-2024-0120775 filed on September 05, 2024, the entire contents of which are incorporated herein by reference.

### FIELD OF THE DISCLOSURE

The present disclosure relates to a method and apparatus for examining bone formation of an alveolar bone and, more particularly, to a method and apparatus for examining bone formation of an alveolar bone by using an intraoral scanner having a tomographic function.

### BACKGROUND

Recently, in order to restore edentulous areas, instances of placing dental implants in the alveolar bone have been on the rise. In order to place a dental implant in the alveolar bone, it is necessary to have an alveolar bone consisting of a bone of an appropriate volume and size. A variety of bone regeneration techniques have been developed to establish alveolar bones of appropriate sizes, and the most widely used alveolar bone formation technique is graft bone regeneration (GBR). Graft bone regeneration (GBR) is also called bone grafting, and is a method of filling a bone defect area of the alveolar bone with a bone grafting material (bone filler), inducing bone formation, and forming the alveolar bone into a form that allows for implant placement.

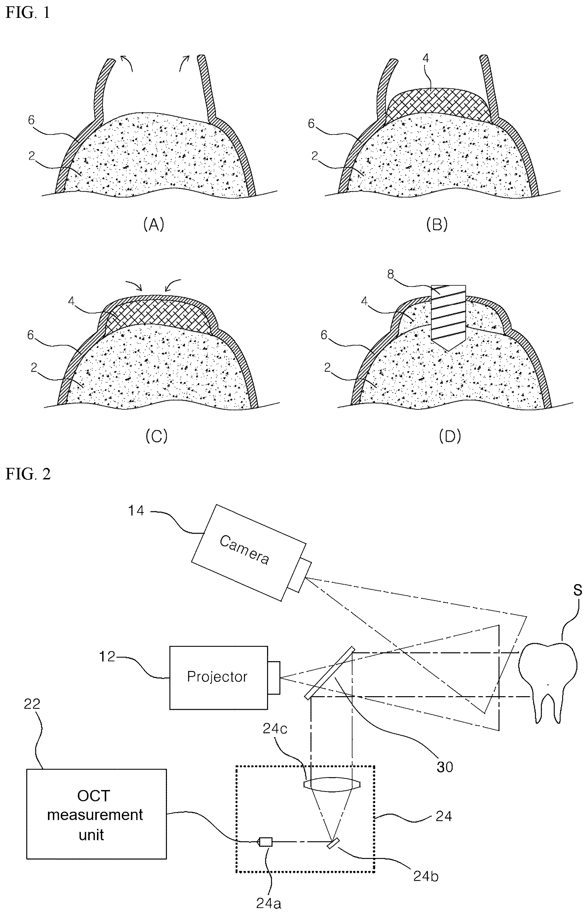

FIG. 1 is a view showing each step of typical graft bone regeneration. As shown in FIG. 1, in the typical graft bone regeneration, the alveolar bone 2 is opened by incising the gums 6 surrounding the alveolar bone 2 where graft bone regeneration is to be performed (A in FIG. 1). Next, the opened alveolar bone 2 is filled with a bone grafting material (also called a "bone filler") 4 (B in FIG. 1), and the gums 6 are sutured to cover the bone grafting material 4 (C in FIG. 1). When the filled bone grafting material 4 is sufficiently ossified and the alveolar bone is formed into a form that allows for implant placement, an implant 8 is placed in the formed alveolar bone. As such, implant placement is possible only when a bone grafting material is filled in the bone defect area and the bone, i.e., the alveolar bone, is formed to a level that allows for implant placement.

Furthermore, in order to perform graft bone regeneration and place an implant, it is necessary to check the degree to which the bone grafting material 4 has ossified and the bone, i.e., the alveolar bone 2, has been formed. To check the degree of alveolar bone formation, techniques of estimating bone density from dental CBCT (cone beam computed tomography) images have been attempted, but no practical clinical techniques that can analyze bone density and examine the degree of bone formation at the target location of alveolar bone implantation have been developed.

In addition, a method of measuring ISQ (implant stability quotient) in the resonance method by contacting the top surface of a placed dental implant after the implant is placed, Periotest that measures the reflected signal after striking the implant, IST (implant stability test), and the like are also known, but these are not methods of measuring bone formation but rather methods of measuring the fixation state (stability) of the implant after implant placement.

Therefore, since it was difficult to know the degree of formation of the alveolar bone where the implant would be placed before implant placement, it was hard to determine the timing of implant placement. Accordingly, in general, the reality is that the timing of implant placement is set to as long as several months after bone grafting depending on the age of the implant placement patient, the amount of bone grafting, etc., and a long period of waiting is made to ensure that the bone grafting material is ossified sufficiently. In other words, the timing of implant placement was only determined empirically in the past according to the patient's age, the amount of bone grafting, etc., and there was, in reality, no appropriate method to examine the degree of bone formation of the bone grafting material.

### Prior Art Literature

### Patent Documents

(Patent Document 1) Korean Patent No. 10-2458985

### Non-Patent Documents

(Non-Patent Document 1) Proc. of SPIE Vol. 8914, 2013, DOI: 10.1117/12.2036345, Laura-Cristina Rusu, "Time Domain Optical Coherence Tomography Investigation of Bone Matrix Interface in Rat Femurs."
(Non-Patent Document 2) Proc. of SPIE Vol. 8925, 2014, DOI: 10.1117/12.2045849, Laura-Cristina RUSU et al. "Different Matrix Evaluation for the Bone Regeneration of Rats Femours using Time Domain Optical Coherence Tomography."

### SUMMARY OF THE DISCLOSURE

### TECHNICAL OBJECTS

It is an object of the present disclosure to provide a method and apparatus for examining bone formation of an alveolar bone, which can examine the state of alveolar bone formation of a bone grafting material, by using an intraoral scanner having a tomographic function.

It is another object of the present disclosure to provide a method and apparatus for examining bone formation, which support the timing of implant placement to be determinable by examining the bone density of an alveolar bone formation area at an implant placement target location before implant placement.

It is yet another object of the present disclosure to provide a method and apparatus for examining bone formation, which can increase the success rate of implant placement and minimize patient discomfort through the early placement of an implant by examining the degree of bone formation of an alveolar bone and allowing the implant to be placed at an appropriate time.

### TECHNICAL SOLUTION

In order to achieve the above objects, the present disclosure provides a method of examining bone formation of an alveolar bone using an intraoral scanner having a tomographic function, wherein the intraoral scanner includes a surface shape acquisition optical system 12 and 14 for obtaining a surface shape image of an oral structure S and an internal cross-section acquisition optical system 22 and 24 for obtaining an internal cross-sectional image of the oral structure S, and the method includes filling an alveolar bone 2 requiring bone grafting with a bone grafting material 4, suturing gums 6 so as to cover the bone grafting material 4, and then obtaining a surface shape image of a part where an alveolar bone is formed using the surface shape acquisition optical system 12 and 14; obtaining an internal cross-sectional image showing a state of the alveolar bone 2 and the bone grafting material 4 by using the internal cross-section acquisition optical system 22 and 24 at a predetermined location 6a of the obtained surface shape image; obtaining an internal cross-sectional image showing the state of the alveolar bone 2 and the bone grafting material 4 at a corresponding location by using the internal cross-section acquisition optical system 22 and 24 of the intraoral scanner when a gum surface shape at the location 6a where the internal cross-sectional image was previously obtained is detected while obtaining gum surface shapes by moving the intraoral scanner after a predetermined period has elapsed; and comparing the internal cross-sectional images obtained at a predetermined time interval.

Furthermore, the present disclosure provides an apparatus for examining bone formation including an intraoral scanner main body 50 in which a surface shape acquisition optical system 12 and 14 for obtaining a surface shape image of an oral structure S and an internal cross-section acquisition optical system 22 and 24 for obtaining an internal cross-sectional image of the oral structure S are housed and in which an opening 52 that passes a shape measurement light irradiated onto a surface of the oral structure S, a layer measurement light irradiated onto an inside of the oral structure S, and a reflected light reflected off the oral structure S is formed; and a press window 60 mounted in the opening 52 of the intraoral scanner main body 50 and configured to transmit the shape measurement light, the layer measurement light, and a signal light and to increase a penetration depth of the layer measurement light by contacting the surface of the oral structure S and pressing against the surface of the oral structure S.

### EFFECTS OF THE DISCLOSURE

According to the method and apparatus for examining the bone formation of an alveolar bone in accordance with the present disclosure, the success rate of implant placement can be increased and patient discomfort can be minimized through the early placement of an implant by examining the alveolar bone formation state of the bone grafting material and allowing the implant to be placed at an appropriate time by using the intraoral scanner having a tomographic function.

### BRIEF DESCRIPTION OF THE DRAWINGS

FIG. 1 is a view showing each step of typical graft bone regeneration;
FIG. 2 is a diagram showing the configuration of an intraoral scanner having a tomographic function that can be used in the present disclosure;
FIG. 3 is a photograph showing a surface shape image of the part where an alveolar bone is formed;
FIG. 4 is an internal cross-sectional image showing the state in which an alveolar bone is formed under the gums; and
FIGS. 5 and 6 are views showing an apparatus for examining bone formation according to embodiments of the present disclosure.

### DETAILED DESCRIPTION OF THE PREFERRED EMBODIMENTS

Hereinafter, the present disclosure will be described in detail with reference to the accompanying drawings.

FIG. 2 is a diagram showing the configuration of an intraoral scanner having a tomographic function that can be used in the present disclosure. As shown in FIG. 2, the intraoral scanner having a tomographic function that can be used in the present disclosure includes a surface shape acquisition optical system 12 and 14 for obtaining surface shape images of an oral structure S such as teeth, gums, and alveolar bone, and an internal cross-section acquisition optical system 22 and 24 for detecting reflected light (scattered light) reflected off the inside of the oral structure S, specifically, each layer inside the oral structure S and for obtaining internal cross-sectional images of the oral structure S.

For example, the intraoral scanner having a tomographic function according to the present disclosure may include the surface shape acquisition optical system including a shape measurement light projector 12 and a shape measurement camera 14; the internal cross-section acquisition optical system including an OCT (optical coherence tomography) measurement unit 22 and an OCT scan probe 24; and a beam splitter 30.

The shape measurement light projector 12 emits a shape measurement light for obtaining a shape image of the oral structure S such as teeth, gums, and alveolar bone. As the shape measurement light, any measurement light that can obtain a shape image of the oral structure S may be used without limitations, and preferably a visible light, for example, a visible light with a wavelength of 400 to 700 nm, may be used.

The shape measurement camera 14 is a device that obtains a surface shape image of the oral structure S by detecting the reflected light formed by the shape measurement light being reflected off the surface of the oral structure S, and includes a typical image sensor.

In operation, a shape measurement light is outputted from the shape measurement light projector 12, the outputted shape measurement light passes through the beam splitter 30 and is then irradiated onto the oral structure S, the reflected light reflected off the oral structure S is detected by the shape measurement camera 14, and a surface shape image of the oral structure S is thus obtained. At this time, the two-dimensional image of the oral structure S obtained with the shape measurement camera 14 may be converted into a three-dimensional image by using triangulation or the like.

The OCT measurement unit 22 transmits a layer measurement light (e.g., a near-infrared light) through the oral structure S, and detects reflected light (scattered light) reflected off the inside of the oral structure S, specifically, each layer inside the oral structure S, thereby obtaining an internal cross-sectional image of the oral structure S. The OCT measurement unit 22 is a device that obtains layer information inside an object by using the coherence properties of the layer measurement light. For example, the layer measurement light may be a broadband low-coherence light having a short coherence distance, and may preferably be a near-infrared light, specifically, a near-infrared light having a wavelength of 750 to 1500 nm.

The OCT scan probe 24 is a device that directs the layer measurement light emitted from the OCT measurement unit 22 to a desired location on the oral structure S, and transfers the reflected light reflected off the oral structure S to the OCT measurement unit 22. The OCT scan probe 24 may include a collimator 24a that focuses the layer measurement light and its reflected light; a reflective mirror 24b that reflects the focused layer measurement light to a desired imaging location of the oral structure S and transfers the reflected light reflected off the oral structure S to the collimator 24a; and an objective lens 24c that focuses the measurement light reflected off the reflective mirror 24b to the desired imaging location of the oral structure S. Here, as the reflective mirror 24b, a micro-electro-mechanical system (MEMS) mirror capable of sequentially scanning the imaging locations on the oral structure S by adjusting the reflection angle of the layer measurement light may be used. For example, the reflective mirror 24b rotates about two axes (e.g., the x-axis and y-axis in an orthogonal relationship) and scans sequentially the plane on which the oral structure S is located, and the layer measurement light is irradiated into the oral structure S in a direction perpendicular to the plane (the z-axis direction, orthogonal to the x-axis and y-axis), and a three-dimensional layer image of the oral structure S can thus be obtained.

The beam splitter 30 is a device that superimposes the optical paths of the shape measurement light emitted from the shape measurement light projector 12 and of the layer measurement light emitted from the OCT scan probe 24, and superimposes the surface shape acquisition optical system formed by the shape measurement light projector 12 and the shape measurement camera 14 and the internal cross-section acquisition optical system formed by the OCT measurement unit 22 and the OCT scan probe 24. For example, as shown in FIG. 2, the beam splitter 30 may be a dichroic mirror 30 that irradiates the oral structure S with the shape measurement light and the layer measurement light by transmitting the shape measurement light emitted from the shape measurement light projector 12 and reflecting the layer measurement light emitted from the OCT scan probe 24, and separates and transfers each reflected light to the shape acquisition optical system (specifically, the shape measurement camera 14) and the layer acquisition optical system (specifically, the OCT measurement unit 22). As shown in FIG. 2, if the dichroic mirror 30, which reflects the layer measurement light but transmits the shape measurement light, is positioned at a location through which the shape measurement light from the shape measurement light projector 12 passes and which is not included in the FOV (field of view) region of the shape measurement camera 14, an integrated optical system can be formed in which the measurement areas of each of the shape acquisition optical system and the layer acquisition optical system, i.e., ROIs (regions of interest), are superimposed. Therefore, the dichroic mirror 30 makes it possible to obtain both an external surface shape image and an internal cross-sectional image of the oral structure S by superimposing the shape measurement light and the layer measurement light and directing them to the oral structure S.

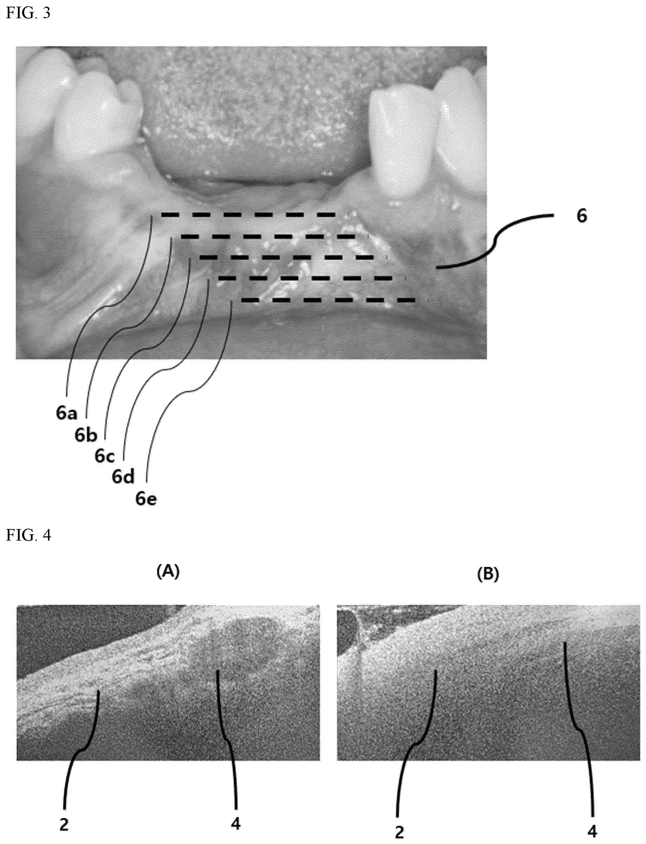

Next, a method of examining bone formation of an alveolar bone according to one embodiment of the present disclosure will be described with reference to FIGS. 2 to 4. FIG. 3 is a photograph showing the gums, i.e., the surface shape image, of the part where an alveolar bone is formed, and FIG. 4 is a photograph showing an internal cross-sectional image showing the state in which an alveolar bone is formed under the gums.

In order to examine the bone formation of the alveolar bone according to the present disclosure, a bone grafting material 4 is filled into the alveolar bone 2 requiring bone grafting, the gums 6 are sutured to cover the bone grafting material 4, and then, a surface shape image of the part where the alveolar bone is formed, specifically, a surface shape image of the gums 6 (see FIG. 3) is obtained using the surface shape acquisition optical system 12 and 14 of the intraoral scanner having a tomographic function as shown in FIG. 2.

At predetermined locations 6a, 6b, 6c, 6d, and 6e of the surface shape image thus obtained, an internal cross-sectional image (see A in FIG. 4) showing the state of the alveolar bone 2 and the bone grafting material 4 under the gums 6 is obtained using the internal cross-section acquisition optical system 22 and 24 of the intraoral scanner having a tomographic function as shown in FIG. 2.

Here, the surface shape image (e.g., FIG. 3) obtained using the shape acquisition optical system of the intraoral scanner serves as a guide image for determining the locations 6a, 6b, 6c, 6d, and 6e where the internal cross-sectional images are obtained. In a specific description of this, when a gum surface shape of a predetermined shape is obtained while obtaining gum surface shapes by moving the intraoral scanner (i.e., progressive scan), an internal cross-sectional image (e.g., A in FIG. 4) showing the state of the alveolar bone 2 and the bone grafting material 4 is obtained at the corresponding location (e.g., location 6a in FIG. 3) by using the layer acquisition optical system of the intraoral scanner. At this time, if necessary, while obtaining a gum surface shape at another location by further moving the intraoral scanner, an internal cross-sectional image showing the state of the alveolar bone 2 and the bone grafting material 4 can be further obtained at another location (e.g., location 6b in FIG. 3) by using the internal cross-section acquisition optical system 22 and 24 of the intraoral scanner.

After a predetermined period (e.g., three months) has elapsed since obtaining the internal cross-sectional image after the bone grafting material 4 was filled in this way, an internal cross-sectional image (e.g., B in FIG. 4) showing the state of the alveolar bone 2 and the bone grafting material 4 at the corresponding location is obtained by using the internal cross-section acquisition optical system 22 and 24 of the intraoral scanner when a gum surface shape at the location where the internal cross-sectional image was previously obtained (e.g., location 6a in FIG. 3) is detected (e.g., FIG. 3) while obtaining gum surface shapes by moving the intraoral scanner again.

The surface shape of the patient's gums can be considered to be substantially the same during the bone formation process. Therefore, if a surface shape image of the part where the alveolar bone is formed is obtained using the surface shape acquisition optical system of the intraoral scanner, and internal cross-sectional images (e.g., A and B in FIG. 4) are obtained at the location where the same surface image was obtained after a predetermined time interval, then the change in the internal cross-sectional images (e.g., A and B in FIG. 4) can be observed at the same location.

Therefore, the surface shape image obtained using the surface shape acquisition optical system 12 and 14 of the intraoral scanner serves as a "guide image for determining (tracking) a cross-section acquisition location," which determines, i.e., tracks, the acquisition location of the internal cross-sectional image obtained using the internal cross-section acquisition optical system 22 and 24 of the intraoral scanner. Further, since the internal cross-sectional images (e.g., A and B in FIG. 4) obtained at a predetermined time interval using the internal cross-section acquisition optical system of the intraoral scanner show the bone formation state of the bone grafting material 4, the degree of bone formation of the alveolar bone can be examined by comparing the internal cross-sectional images (e.g., A and B in FIG. 4) obtained at a predetermined time interval. In other words, by comparing the internal cross-sectional images, it is possible to determine whether the degree of bone formation of the bone grafting material 4 is suitable for implant placement.

For example, the image of the alveolar bone 2 in black and the image of the bone grafting material 4 are clearly distinguished in the initial internal cross-sectional image shown in A of FIG. 4, but the image of the bone grafting material 4 has changed to a color similar to the surroundings in the internal cross-sectional image shown in B of FIG. 4 after three months, confirming that ossification has progressed. Here, since the initial bone grafting material 4 is in the form of agglomerated powder, reflection occurs on the surface of each powder particle and an image of a different color from the surroundings is obtained, but when the bone grafting material 4 has ossified, the powder aggregates and the boundary surfaces disappear, resulting in an image of a color similar to the surroundings.

The method of examining bone formation of an alveolar bone according to the present disclosure is a method of obtaining information on the degree of bone formation of the bone grafting material 4, and may be performed by a control unit (not shown) of an intraoral scanner with a tomographic function. For example, the control unit of the intraoral scanner having a tomographic function may obtain the surface shape images and the internal cross-sectional images by controlling the surface shape acquisition optical system 12 and 14 and the internal cross-section acquisition optical system 22 and 24, set the location for obtaining the internal cross-sectional image by comparing the equality or difference of the surface shape images and the internal cross-sectional images, and obtain bone formation information of the alveolar bone by detecting a change in the internal cross-sectional image.

The method of examining bone formation according to the present disclosure may be performed using the intraoral scanner having a typical tomographic function as shown in FIG. 2. However, since the internal cross-section acquisition optical system of the typical intraoral scanner has a small tomographic depth, a cross-sectional image of the bone grafting material 4 under the gums 6 cannot be obtained sufficiently if the gums 6 (see C in FIG. 1) are thick, for example. Therefore, the present disclosure provides an apparatus for examining bone formation, particularly suitable for the method of examining bone formation according to the present disclosure.

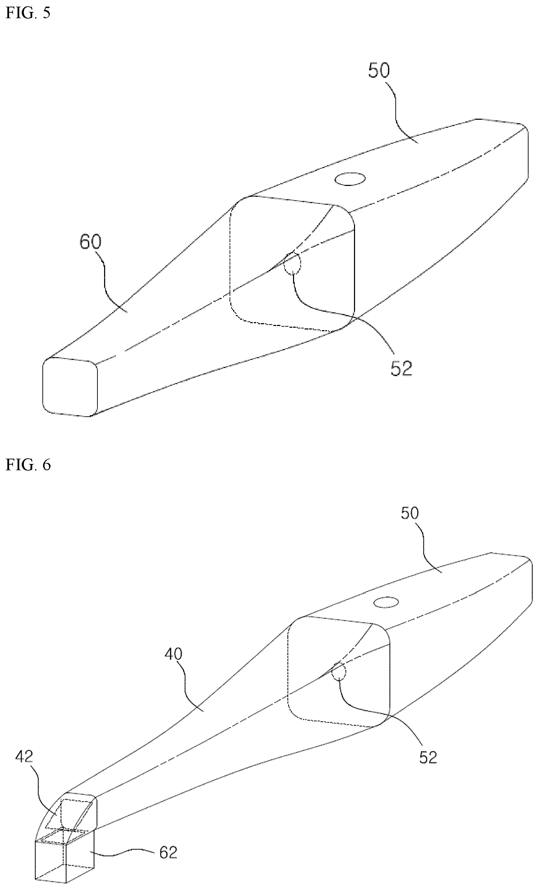

FIG. 5 is a view showing an apparatus for examining bone formation according to an embodiment of the present disclosure. As shown in FIG. 5, the apparatus for examining bone formation according to an embodiment of the present disclosure includes an intraoral scanner main body 50 in which a surface shape acquisition optical system 12 and 14 for obtaining surface shape images of an oral structure S and an internal cross-section acquisition optical system 22 and 24 for detecting reflected light reflected off each layer inside the oral structure S and obtaining internal cross-sectional images of the oral structure S are housed and in which an opening 52 that passes a shape measurement light irradiated onto the surface of the oral structure S, a layer measurement light irradiated onto the inside of the oral structure S, and the reflected light reflected off the oral structure S is formed; and a press window 60 that is mounted in the opening 52 of the intraoral scanner main body 50, transmits the shape measurement light, the layer measurement light, and a signal light, and can increase the penetration depth of the layer measurement light by contacting the surface of the oral structure S and pressing against the oral structure S, specifically, the gums 6.

The press window 60 may be made of a material that can transmit the shape measurement light, the layer measurement light, and the signal light and can increase the penetration depth of the layer measurement light by contacting the surface of the oral structure S and pressing against the surface of the oral structure S, and may be made of, for example, glass, light-transmitting plastic, or the like.

When the surface of the oral structure S is pressed using the press window 60, the surface thickness of the oral structure S gets thinner, and thus, the alveolar bone formation inside the oral structure S can be examined more easily.

FIG. 6 is a view showing an apparatus for examining bone formation according to another embodiment of the present disclosure. The apparatus for examining bone formation according to the embodiment shown in FIG. 6 has the same configuration as the apparatus for examining bone formation shown in FIG. 5, except that an oral tip 40, on which a reflective mirror 42 for guiding the shape measurement light and the layer measurement light to the inside of the oral cavity is mounted, is attached to the intraoral scanner main body 50, and a press window 62 is mounted on a shape measurement light, layer measurement light, and signal light transmission area at the distal end of the oral tip 40. In the embodiment shown in FIG. 6, the press window 62 has a rectangular parallelepiped shape according to the shape of the distal end of the oral tip 40.

In the apparatus for examining bone formation of the present disclosure, the form of the press windows 60 and 62 can be modified in various ways. The press window 60 may have not only the form of a rectangular prism as shown in FIGS. 5 and 6 but also the form of a triangular prism, a cylinder, etc., and the width and thickness can be set appropriately according to the location of the oral structure S to be observed.

Although the present disclosure has been described above with reference to the accompanying drawings and example embodiments, the present disclosure is not limited to what is shown in the drawings and the embodiments described above. In the following claims, reference numerals are indicated to aid understanding, but the scope of the following claims should not be limited to what is shown by the reference numerals and in the drawings and should be construed to encompass all modifications, and equivalent constructions and functions of the example embodiments.

## Claims

1. A method of examining bone formation of an alveolar bone using an intraoral scanner having a tomographic function, wherein the intraoral scanner comprises a surface shape acquisition optical system (12 and 14) for obtaining a surface shape image of an oral structure (S) and an internal cross-section acquisition optical system (22 and 24) for obtaining an internal cross-sectional image of the oral structure (S), the method comprising:
filling an alveolar bone (2) requiring bone grafting with a bone grafting material (4), suturing gums (6) so as to cover the bone grafting material (4), and then obtaining a surface shape image of a part where an alveolar bone is formed using the surface shape acquisition optical system (12 and 14);
obtaining an internal cross-sectional image showing a state of the alveolar bone (2) and the bone grafting material (4) by using the internal cross-section acquisition optical system (22 and 24) at a predetermined location (6a) of the obtained surface shape image;
obtaining an internal cross-sectional image showing the state of the alveolar bone (2) and the bone grafting material (4) at a corresponding location by using the internal cross-section acquisition optical system (22 and 24) of the intraoral scanner when a gum surface shape at the location (6a) where the internal cross-sectional image was previously obtained is detected while obtaining gum surface shapes by moving the intraoral scanner after a predetermined period has elapsed; and
comparing the internal cross-sectional images obtained at a predetermined time interval.

2. The method of claim 1, wherein the surface shape image of the part where the alveolar bone is formed is a surface shape image of the gums (6).

3. The method of claim 1, further comprising:
obtaining an internal cross-sectional image showing the state of the alveolar bone (2) and the bone grafting material (4) at another location (6b) by using the internal cross-section acquisition optical system (22 and 24) of the intraoral scanner while obtaining a gum surface shape at another location by further moving the intraoral scanner.

4. The method of claim 1, wherein by the comparing the internal cross-sectional images, it is determined whether a degree of bone formation of the bone grafting material (4) is suitable for implant placement.

5. The method of claim 1, wherein the method is performed by a control unit of the intraoral scanner having a tomographic function.

6. An apparatus for examining bone formation comprising:
an intraoral scanner main body (50) in which a surface shape acquisition optical system (12 and 14) for obtaining a surface shape image of an oral structure (S) and an internal cross-section acquisition optical system (22 and 24) for obtaining an internal cross-sectional image of the oral structure (S) are housed and in which an opening (52) that passes a shape measurement light irradiated onto a surface of the oral structure (S), a layer measurement light irradiated onto an inside of the oral structure (S), and a reflected light reflected off the oral structure (S) is formed; and
a press window (60) mounted in the opening (52) of the intraoral scanner main body (50), and configured to transmit the shape measurement light, the layer measurement light, and a signal light and to increase a penetration depth of the layer measurement light by contacting the surface of the oral structure (S) and pressing against the surface of the oral structure (S).

7. The apparatus of claim 6, wherein the surface of the oral structure (S) is gums (6).
